# EUROPEAN PATENT SPECIFICATION

(11) **EP 0 972 840 B1**
(45) Date of publication and mention of the grant of the patent: **19.05.2004**
(21) Application number: 99500099.9
(22) Date of filing: 10.06.1999
(51) Int. Cl.: C12N 15/86, C12N 15/41, A61K 39/295, A61K 39/275, A61K 39/125

(54) **New attenuated myxoma recombinant virus and its use in the preparation of mixed vaccines against myxomatosis and rabbit hemorrhagic disease**
Attenuierte rekombinante Myxoma Viren und deren Verwendungen in der Herstellung von gemischten Impfstoffen gegen Myxomatosis und Hämorrhagie Krankheit bei Kaninchen
Virus myxoma recombinant attenué, et son utilisation dans la préparation de vaccins mixtes contre la myxomatose et la maladie de l'hémorrhagie des lapins

(30) Priority: 10.06.1998 ES 9801219
(43) Date of publication of application: 19.01.2000
(73) Proprietor: Fundacion Para el Estudio y Defensa de la Naturaleza y de la Caza, 28039 Madrid (ES); INSTITUTO NACIONAL DE INVESTIGACION Y TECNOLOGIA, AGRARIA Y ALIMENTARIA, E-28003 Madrid (ES)
(72) Inventor: Barcena del Riego, Juan, 28006 Madrid (ES); Blasco Lozano, Rafael, 28863 Cobena (ES); Morales Camarzana, Monica, 28041 Madrid (ES); Pages Mante, Albert, 17853 Tortella (ES); Parra Fernandez, Francisco, 33006 Oviedo (ES); Sanchez Vizcaino, Jose Manuel, 28150 Valdetorres del Jarama (ES); Torres Trillo, Juan Maria, 28100 Alcobendas (ES); Vazquez Ruiz, Belen, 28037 Madrid (ES)
(74) Representative: SUGRANES - VERDONCES - FERREGÜELA

(56) References cited:
- FR-A- 2 736 358
- BERTAGNOLI STEPHANE ET AL: "Protection against myxomatosis and rabbit viral hemorrhagic disease with recombinant myxoma viruses expressing rabbit hemorrhagic disease virus capsid protein." JOURNAL OF VIROLOGY, vol. 70, no. 8, 1996, pages 5061-5066, XP002169078 ISSN: 0022-538X
- JACKSON R J ET AL: "A MYXOMA VIRUS INTERGENIC TRANSIENT DOMINANT SELECTION VECTOR" JOURNAL OF GENERAL VIROLOGY,GB,SOCIETY FOR GENERAL MICROBIOLOGY, READING, vol. 73, 1 December 1992 (1992-12-01), pages 3241-3245, XP002002210 ISSN: 0022-1317
- KERR P J ET AL: "Myxoma virus as a vaccine vector for rabbits: antibody levels to influenza virus haemagglutinin presented by a recombinant myxoma virus" VACCINE,GB,BUTTERWORTH SCIENTIFIC. GUILDFORD, vol. 13, no. 17, 1 December 1995 (1995-12-01), pages 1722-1726, XP004057403 ISSN: 0264-410X
- JACKSON RONALD J ET AL: "Construction of recombinant myxoma viruses expressing foreign genes from different intergenic sites without associated attenuation." JOURNAL OF GENERAL VIROLOGY, vol. 77, no. 7, 1996, pages 1569-1575, XP002169080 ISSN: 0022-1317
- BARCENA JUAN ET AL: "Horizontal transmissible protection against myxomatosis and rabbit hemorrhagic disease by using a recombinant myxoma virus." JOURNAL OF VIROLOGY, vol. 74, no. 3, February 2000 (2000-02), pages 1114-1123, XP002169081 ISSN: 0022-538X
- BARCENA J. ET AL: "isolation of an attenuated myxoma virus field strain that can confer protection against myxomatosis on contacts of vaccinates" ARCH. VIROL, vol. 145, no. 4, 2000, pages 759-771, XP001002748

## Description

### Technical Field of the Invention

The present invention refers to a new life recombinant myxoma virus, attenuated and having capacity of a controlled transmission and comprising within its genome a nucleotide sequence which encodes and expresses an antigen protein of the Rabbit Hemorrhagic Disease Virus (RHDV), and the use of said new virus in the preparation of combined vaccines against myxomatosis and rabbit hemorrhagic disease, wherein the vaccines have a capacity of self-propagating in controlled manner. The present invention also refers to a method for obtaining a recombinant myxoma virus having a controlled transmission capacity and to its use in the preparation of combined vaccines for leporids.

### Previous State of the Art

The two main diseases that affect rabbits are myxomatosis (MV) and the hemorrhagic viral disease (RHDV), diseases that are causing serious losses in the production sector of this animal species. Likewise, and given the important role that wild rabbits play in the equilibrium of certain ecosystems, fundamentally the Iberian, these two diseases are creating serious problems in the said ecosystems, endangering not only the survival of the rabbit, but also their natural predators (eagle, lynx, etc.)

The virus of the rabbit myxomatosis belongs to the *poxvirus* family, which constitute one of the viral groups of greatest genetic and biochemical complexity. The launching of a prophylactic sanitary system against myxomatosis is extremely complicated due to its epidemiological peculiarities, such as its transmission is performed fundamentally by arthropod vectors, principally fleas and mosquitoes.

The Rabbit Hemorrhagic Disease Virus (RHDV), belongs to the *calicivirus* family and is of a size ten times smaller than the myxomatosis virus. The virus exhibit a single pathologic level capable of killing a rabbit in only 20 hours. Given its lethality, there is no known natural means by which the virus could immunize rabbits. The disease is spread by, in addition to the aforesaid vectors, via aerogenic and alimentary routes and by physical contact, among other means.

The RHDV structural protein VP60 is sufficient in itself to generate an immune response that protects rabbits against the disease. In the Spanish patent application ES-A1-2.080.024, priority for the European patent application EP-A2-0 704 529, the obtention of vaccines against RHDV prepared from a recombinant *baculovirus* containing a nucleotide sequence that expresses the antigen protein VP60 is described.

In the French patent application FR-A1-2.736.358 a recombinant *myxoma* virus is described that contains a nucleotide sequence which produces said antigen protein VP60 and its use in the preparation of combined vaccines against myxomatosis and rabbit hemorrhagic disease. However, given that the recombinant virus described in said patent application lacks transmission capacity, the vaccines obtained from it are not self-propagating and need to be administered individually to each animal that is to be immunized. This is a serious problem for its use in industrial exploitations involving a large number of animals, which must be treated individually, and in no way resolves the immunization problem of wild rabbit populations, since its final utilization in the field would necessitate the previous capture of a large number of specimens, which is not viable in practice.

Accordingly, although on the one hand, simple vaccines against myxomatosis and RHD exist, and on the other, there exists a non-self-propagating combined vaccine against myxomatosis and RHD which must be administered individually to each animal to be immunized, at the current time there is no combined vaccine of said type which contains a substantial attenuation level and is capable of self-propagating in a controlled manner in stock exploitation, and allowing its efficient employment in the immunization of wild rabbit populations.

### Object of the Invention

The object of the present invention is a new recombinant *myxoma* virus, comprising a nucleotide sequence capable of expressing RHDV antigen proteins, exhibiting attenuated virulence, and a controlled self-propagating capacity.

A further object of the present invention is the use of said recombinant virus in the preparation of combined vaccines against myxomatosis and hemorrhagic rabbit diseases, as well as the vaccines obtained from said virus.

Also falling within the objects of the present invention is a new method for obtaining a recombinant *myxoma* virus possessing attenuated virulence and a controlled self-propagating capacity.

### Description of the invention

The new *myxoma* virus, object of the present invention, has been deposited in the Collection Nationale de Cultures de Microorganismes at the Institut Pasteur (CNCM), in accordance with the conditions of the Budapest Treaty on the International Recognition of Deposit of Microorganisms for the Purposes of Patent Procedure (1977), and has been assigned deposit number I-1990 and deposit date 6^{th} March, 1998.

Said new virus has been designated by the inventors as 6918-VP60-T2, for which reason throughout the present description the terms I-1990 and 6918-VP60-T2 will be applied indiscriminately.

The new I-1990 virus is constructed from a myxoma virus strain, designated by the inventors 6918, selected for its low pathogenicity and its controlled propagation capacity, in which the following elements have been inserted in its genome, via conventional DNA recombinant techniques:
a) a nucleotide sequence capable of expressing the protein VP60,
b) a promoter controlling the expression, and
c) a nucleotide sequence encoding an antigen polypeptide unrelated to VP60, the detection of which allows to differentiate the vaccinated animals from those which are not.

The 6918 strain was selected from a myxomatosis viral strain collection obtained from naturally infected wild animals. The characteristics of the selected strain are summarized below:

In the studied dosages (10²-10⁵ plaque forming units/animal), the 6918 *myxoma* strain possesses the characteristic of producing small myxomas (less than 2 mm in diameter) in the inoculation point and in the ears, all of this without producing any mortality nor apparent disease in the inoculated animals.

The 6918 strain exhibited during three consecutive transmission steps the mortality and transmissibility shown in the following table:

**Table 1.**

| Mortality and transmissibility of the 6918 strain. | | | | |
|---|---|---|---|---|
| | Step 0 | Step 1 | Step 2 | Step |
| Transmissibility | 100 | 60 | 20 | 0 |
| Mortality | 0 | 0 | 0 | 0 |

This is to say, the 6918 strain exhibited 0% mortality, its transmission capacity was 100% in the first transmission step, and in the succeeding transmission steps this decreased in such a manner that in transmission step 3 its transmission capacity was null.

The cited 6918 strain exhibits habitual properties and characteristics regarding its growth and cultivation.

The nucleotide sequence capable of producing the VP60 protein is obtained from the isolate AST/89, described in the previously mentioned Spanish patent application ES-A1-2.080.024, and has the access number Z49271 in the EMBL database.

The promoter that controls production of said nucleotide sequence is the synthetic promoter early/ late optimized for expression of genes in different *poxvirus* as described by Blasco, R. and B. Moss, Gene. 158, 157-162, (1995) and by Bárcena, J. And R. Blasco, Virology, in press, (1998).

The nucleotide sequence for detection which encodes an antigen polypeptide unrelated to VP60 corresponds to the sequence of an epitope, designated DA3, of the N protein of the porcine gastroenteritis virus.

The cited nucleotide sequences and the promoter are inserted into the intergenic region situated between the thymidine kinase gene and the MF8a gene, in such a manner that the insertion respects the regulatory sequences of the TK gene, as previously described by Jackson et al., J. Gen. Virol. 73:323-328 (1992), and the sequences preceding the ATG start codon of the MF8a gene.

The chosen intergenic zone does not affect the replication capacity or transmission capacity of the virus, which is especially advantageous since it allows to maintain the transmission capacity of the original *myxoma* strain 6918.

The new virus I-1990 object of the invention allows the obtaining of live combined vaccines, effective against myxomatosis and rabbit hemorrhagic disease, exhibiting a limited transmission capacity for a maximum of three transmissions, guaranteeing on the one hand their self-propagating capacity and on the other that said self-propagation does not occur in an uncontrolled manner, leading eventually to sanitary or ecological risks.

Said vaccines may be prepared using conventional vaccine preparation techniques based on attenuated live virus, which are well-known and commonly employed by experts in the field, in such a manner that they contain an efficient viral dosage, which may be considered to be within the range of 10² and 10⁵ plaque forming units (pfu) /animal, and may be administered via intradermal, subcutaneous or oral routes.

The mentioned vaccines exhibit evident advantages with regard to those previously described, since they allow inoculation of large rabbit populations without the need to recur to individualized vaccination of each animal, and moreover, their self-propagation capacity disappears after three transmissions at most, which guarantees against an uncontrolled expansion of the vaccinating virus. Furthermore, the vaccines which are object of the invention efficiently resolve the problem of vaccination of wild rabbits, since vaccination of an aliquot part of the wild population and the resulting controlled propagation of the vaccinal virus, via the same infectious vectors of the myxoma virus, promotes a generalized vaccination in the wild population, at least in a considerable proportion, without risk of the virus extending in an uncontrolled manner.

The results obtained with the new virus I-1990 permits to establish that the method used for its construction is a suitable strategy for the development of other combined vaccine variants for immunization of leporids against myxomatosis and other types of infectious diseases or other varieties of rabbit hemorrhagic disease.

For this reason it must be considered within the object of the present invention, a new method for obtaining recombinant *myxoma* virus, attenuated and capable of self-propagating in a controlled manner, and the vaccines obtained thereby, consisting of the following essential steps:
a) selecting and isolating a myxoma virus that exhibits a null or highly reduced mortality and which possesses a transmission capacity of no greater than three transmission steps,
b) inserting within an intergenic region of its genome, by use of conventional DNA recombinant techniques, one or more nucleotide sequences capable of expressing polypeptides or proteins antigens from microorganisms that produce infectious diseases in rabbits, and a promoter controlling such expression, and wherein the insertion is not affecting the transmission capacity of the virus,
c) cloning and replicating the obtained virus.

In a preferred manner, the nucleotide sequences are inserted in the intergenic region situated between the thymidine kinase gene and the MF8a gene, in such a manner that the insertion respects the TK gene regulating sequences and the sequences previous to the ATG start codon of the MF8a gene.

Further, in a preferred manner a second nucleotide sequence encoding an antigen polypeptide, unrelated to the antigen polypeptide or protein, the detection of which allows differentiation between vaccinated and non-vaccinated animals.

### Brief description of the Drawings.

Figure 1 shows the diagram of the construction of the plasmids pMYX4 and pMYX4-VP60.
Figure 2 shows the synthetic DNA fragment sequences inserted as markers in the VP60 gene of the RHDV for constructing the versions VP60-T1 (Fig. 2 A) and VP60-T2 (Fig. 2 B).
Figure 3 shows the diagram of the myxoma-VP60 recombinants constructed in the 6918 strain containing three different VP60 versions.
Figure 4 shows the agarose gel for identification via PCR of the recombinants that have incorporated the VP60 gene.
Figure 5 shows an immune-detection on nitrocellulose paper of the expression products of the myxoma-VP60 recombinants using a antiserum specific for RHDV.
Figure 6 shows an immune-detection on nitrocellulose paper of the expression products of the myxoma-VP60 recombinants using a monoclonal specific for the epitope DA3.
Figure 7 shows the anti-myxomatosis antibody titration graphics obtained by ELISA after rabbit vaccination with the different myxoma-VP60 recombinants.
Figure 8 shows the anti-RHDV antibody titration graphics obtained by ELISA after rabbit vaccination with the different myxoma-VP60 recombinants.
Figure 9 shows the anti-DA3 antibody titration graphics obtained by ELISA after rabbit vaccination with the different myxoma-VP60 recombinants.
Figure 10 shows graphically the rate of survival, after infection with virulent virus (myxomatosis or RHDV), of rabbits vaccinated intra-dermally with a single dose (10³ pfu/animal) of the recombinants myxoma VP60.
Figure 11 shows graphically the rate of survival, after infection with virulent virus (myxomatosis or RHDV), of rabbits vaccinated by contact transmission (in absence of fleas) with the recombinant 6918-VP60-T2.
Figure 12 shows graphically the rate of survival, after infection with virulent virus (myxomatosis or RHDV), of rabbits vaccinated by flea mediated transmission (in absence of contact) with the recombinant 6918-VP60-T2.

### Detailed description of the preferred embodiment

The detailed description that follows below is expounded with the aim of providing a sufficiently clear and complete description of the present invention to the expert in the field, but it must not be considered to be a limitation of the essential aspects of the object of the invention, as they have been expounded in the previous paragraphs of this description.

In first place and with the aim of obtaining a non pathogenic *myxoma* strain which maintained its rabbit to rabbit transmission capacity, a collection of myxomatosis strains isolated from different Spanish areas was studied and characterized according to their pathogenicity and diffusion. The results enabled the selection of the strain designated 6918 that exhibited characteristics of low pathogenicity, while maintaining a limited diffusion capacity. The 6918 strain exhibited 0% mortality and a contact diffusion of 100%.

Taking into account these results, it was decided to study the stability of the 6918 strain after several transmission steps in wild rabbits (hybrids). The results showed that the 6918 strain remained non pathogenic (0% mortality) after three transmission steps, but finally lost its diffusion capacity. This loss of diffusion capacity characteristic of the 6918 strain after three transmission steps is especially important from the point of view of its field use. When this strain is released into the field, its final destination will be extinction after two or three transmission cycles.

Once the 6918 strain was selected, the myxoma recombinants were constructed, comprising sequences derived from the gene of the VP60 protein of RHD. The system is based on the introduction of the exogenous gene into the *myxoma* virus DNA by homologous recombination. To do this an intermediate plasmid was prepared containing the following elements:
- Flanking sequences, derived from the myxoma virus DNA, to direct the insertion of the exogenous gene.
- A synthetic promoter (early/late) to direct the expression of the exogenous gene.
- Cloning site for insertion of the exogenous gene.
- Genetic element containing a promoter (P7.5) and a selectable gene (*E. coli* gpt).

The flanking sequences are designed so that the insertion of the promoter-gene cassette occurs at a point situated in an intergenic region within the genome of the myxoma virus, in such a manner that it does not affect encoding gene sequences of the myxoma virus. The selected position is between the thymidine kinase gene and the MF8a gene, and the insertion respects the regulating sequences of the TK gene [Jackson et al., J. Gen. Virol. 73:323-328 (1992) ]. At the same time the sequences previous to the ATG start codon of the MF8a gene have been respected.

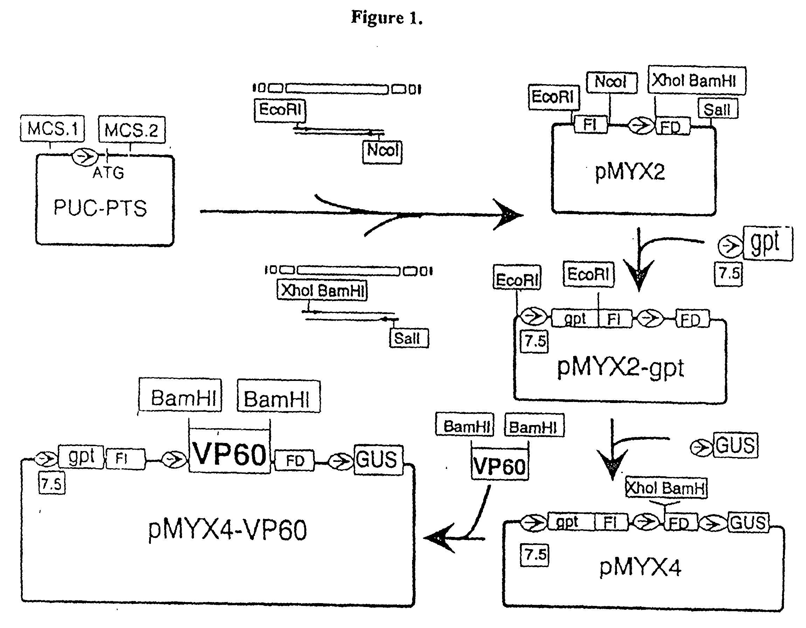

The construction of said intermediate plasmid necessary for the insertion of exogenous genes into the *myxoma* virus DNA has been shown schematically in Figure 1.

The procedure began with the construction of plasmid pUC-PTS, which was obtained by insertion in the BamHI site of the pUC19 plasmid of the DNA sequence generated by hybridization of the following oligonucleotides: and in which the protuberant cohesive ends have been underlined. The DNA sequence contains an early/ late synthetic promoter optimized for gene expression in different *poxvirus* (Blasco, R. and B. Moss, Gene. 158, 157-162, 1995; Bárcena, J. And R. Blasco, Virology, in press, 1998).

For cloning the left flank in the plasmid pUC-PTS the following synthetic oligonucleotides were used: and

Said oligonucleotides were used to perform an amplification by PCR (polymerase chain reaction) using as template DNA from the *myxoma* virus. The corresponding amplified fragment, 529 nucleotides long, contained EcoRI and NcoI sites which were used for its insertion between the EcoRI and NcoI sites in the pUC-PTS plasmid.

Subsequently, for cloning the right flank, the following synthetic oligonucleotides were used: and

Said oligonucleotides were used to perform an amplification by PCR (polymerase chain reaction) using as template DNA from the myxoma virus. The corresponding amplified fragment, 424 nucleotides long, contained XhoI and SalI sites which were used for its insertion between the corresponding sites in the plasmid derived from the pUC-PTS containing the left flank. The resulting plasmid from this cloning was designated pMYX2.

For cloning the selectable gene *gpt*, a fragment containing the 7.5 promoter of the Vaccinia virus and the *E. coli* guanidine phosphoribosyl transferase gene (*gpt*) was amplified by PCR from the pGEM-gpt plasmid (Blasco, R et al., J. Virology 65:4598-4608), using the following synthetic oligonucleotides: and

The amplified fragment contained XhoI and SacI sites which were used for its insertion between the corresponding sites in the pGEM 7-Zf(-) plasmid. Subsequently, a sequence 130 nucleotides long situated between the promoter and the coding sequence of the *gpt* gene was eliminated by digestion with BamHI and BglII and religation. A fragment containing the promoter 7.5-gpt cassette was amplified by PCR from this plasmid using the oligonucleotides:

The corresponding amplified fragment containing the gpt gene and the promoter was digested with EcoRI and was inserted in the EcoRI site of the plasmid pMYX2. The plasmid resulting from this cloning was designated pMYX3.

To clone the visual marker gene GUS, a fragment containing the β-glucuronidase (GUS) gene was inserted in the plasmid pRB21 [Blasco et al., Gene. 158, 157-162, (1995)] under the control of an early/ late synthetic promoter of the vaccine virus, generating the plasmid pRB21-GUS. From this plasmid a XhoI/HindIII fragment was isolated containing the promoter and the GUS gene, which was inserted between the SaI and HindIII sites in the pMYX3 plasmid, creating the pMYX4 plasmid.

In parallel, three different versions of the VP60 protein from the isolate AST/89 of the virus of the rabbit hemorrhagic disease virus (RHDV) were constructed. To do this, the VP60 gene (nucleotides 5305 to 7125 of the isolate AST/89, EMBL database access number Z49271) was inserted in the plasmid pMA91 [Mellor et al., Gene 24: 1-14 (1983)] as has been previously described [Boga et al., J. Gen. Virol. 78: 2315-2318 (1997)]. Within this plasmid the VP60 gene is flanked by restriction sites BglII.

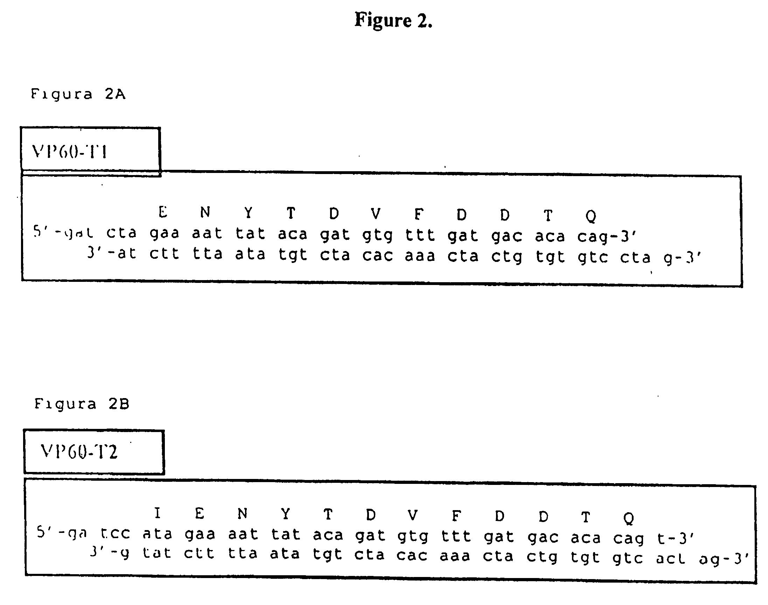

To obtain the VP60-T1 construction, firstly a BglII-SphI fragment of the pMAVP60 plasmid (approx. 360 bp) containing the 5' region of the VP60 gene was inserted into the vector pUCHSV TK [de la Peña and Zasloff, Cell. 50: 613-619 (1987) ], digested with the same enzymes. The resulting recombinant vector digested with BamHI and HindIII was ligated with the BamHI-HindIII fragment (1731 pb) obtained from pT35 cDNA [Boga et al., J. Gen. Virol. 75: 2409-2413 (1994)]. The latter fragment provides the major part of the VP60 coding region. The resulting recombinant vector designated pTKVP60 contains a BglII-HindIII fragment coding the complete VP60 of the RHDV. Finally, to obtain the VP60-T1 construction a synthetic DNA fragment of 39 base pairs (Figure 2A), that encodes the epitope of the nucleoprotein of the porcine transmissible gastroenteritis virus (TGEV) recognized by the monoclonal antibody DA3 [Martin Alonso et al., Virology 188: 168-174 (1992) ], was cloned in the BamHI site of the pTKVP60 (situated within the VP60 gene in position 5394 of the cDNA of the isolate AST/89 of the RHDV). Finally, the vector pTKVP60-DA3 containing the coding region of the epitope in correct orientation was digested with HindIII, the cohesive ends were repaired and a BglII site was generated in the 3'end of the VP60 gene using commercial linkers. The resulting vector, designated pVP60-T1, has a BglII fragment (approx. 2.1 kbp) that contains the complete coding region of the VP60 labeled with the epitope DA3 in an internal region.

The VP60-T2 construction contains, joined to the 3' end of the VP60 gene, a synthetic DNA segment of 42 base pairs of bases that codes for the epitope recognized by the monoclonal antibody DA3 [Martin Alonso et al., Virology 188: 168-174 (1992) ] followed by a translation termination codon (Figure 2B).

The construction was performed according to the following steps:
(1) Cloning of a synthetic DNA segment that codifies the epitope of the monoclonal antibody DA3 in the baculovirus transfer vector to pAcYM1 [Matsura et al., J. Gen. Virol. 68: 1233-1250 (1987) ]. The insertion was performed in the BamHI restriction site (unique in the vector), regenerating the cloning site on the 5' end of the synthetic fragment. In this manner the vector designated pAcYM1-DA3 is obtained.
(2) Amplification by polymerase chain reaction of the VP60 gene with elimination of its termination codon. Said amplification uses the synthetic oligonucleotides 0-5 [Boga et al., J. Gen. Virol. 75: 2409-2413 (1994)] and 3'-Bgl (5' - CCAGTCCGATGAGATCTGACATAGG -3')(SEQ ID NO 11) which add a Bg1II restriction site on both ends, eliminating the termination codon of the VP60 gene.
(3) Cloning of the fragment obtained in the amplification reaction into the cloning vector pGEM-T (Promega Corporation, Madison, WI. USA). In this manner the pGEM-VP60 vector was obtained.
(4) Cloning of the fragment obtained by restriction with BglII of the pGEM-VP60 vector into the pAcYM1-DA3 vector cut with the same enzyme and desphosphorylated. In this manner, a vector is obtained, designated pAcYM1-VP60-DA3, containing the DNA sequence that encodes the DA3 epitope fused to the 3' end of the VP60 gene followed by a translation termination codon. Moreover, the joining of the fragment to the vector pAcYM1-DA3 eliminates the BglII restriction site of the 3' end.
(5) The pAcYM1-VP60-DA3 vector was cleaved with the HindIII restriction endonuclease (situated within the pAcYM1 vector) and BstEII (situated within the VP60 gene), and this resulting restriction fragment was purified and it was inserted into the pTKVP60 vector previously digested with the same restriction endonucleases. In this manner was obtained the plasmid designated pTKVP60-DA3, containing the construction VP60-T2 flanked by BglII-HindIII and which includes the coding sequence of the epitope of the DA3 monoclonal antibody fused at the 3'end of the VP60 gene.

Subsequently, the different versions of the VP60 of rabbit hemorrhagic disease virus were inserted in the plasmid pMYX4.

In the plasmid pMYX4, BamHI fragments containing the different versions of the VP60 protein of the rabbit hemorrhagic disease were inserted. In this manner the following plasmids were obtained: pMYX4-VP60, pMYX4-VP60-T1 and pMYX4-VP60-T2.

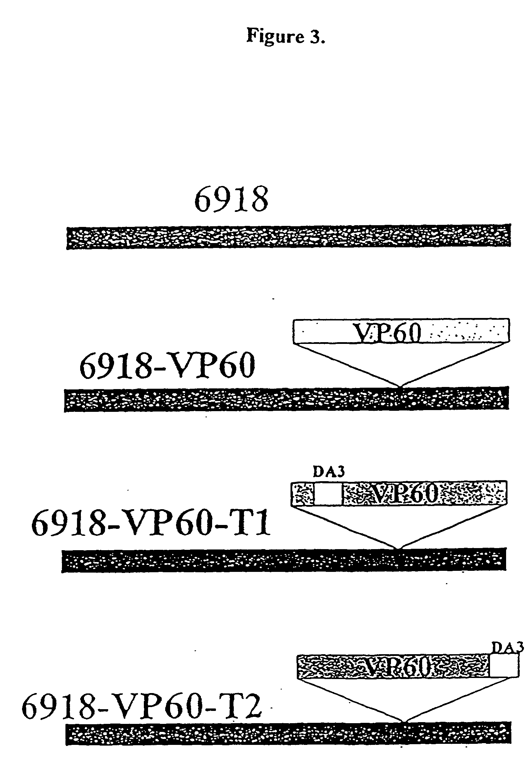

Fig. 3 depicts the different recombinants obtained from the *myxoma* 6918 strain containing the different VP60 versions. The procedure used was identical in all cases. The introduction of the exogenous gene was performed by homologous recombination using strategies developed for vaccinia virus [Earl, P., et al., Current Protocols in Molecular Biology. Wiley-Interscience, New York (1991)]. To do this, SIRC rabbit cornea cells were infected with the strains indicated above, followed by transfection with DNA from the plasmids by the calcium phosphate method. The isolation of the recombinants was performed by the dominant transitory selection method [Falkner, F. G., and B. Moss J. Virol. 64:3108-3111 (1990)] using the *gpt* gene. To do this, the virus progeny of the infection/ transfection was amplified 4 consecutive times in RK-13 cell cultures the presence of micophenolic acid. After this process, a "single" recombinant virus was isolated, containing the exogenous gene, the gpt gene, and the GUS gene. The isolation of this virus was carried out by plaque assay in presence of X-Gluc, to detect the GUS (+) virus. The "single" recombinant viruses contain repeated sequences, which means they are unstable, leading to a second recombination event by which the parental virus or a double recombinant appear. The process was assisted by 2-3 cell culture passages of the "single" recombinant in the absence of selection, followed by isolation of different clones by plaque assay in the absence of selection. Finally, the viruses which had incorporated the VP60 gene were identified by PCR using a primer corresponding to the left flanking sequence and another primer corresponding to the internal VP60 sequence

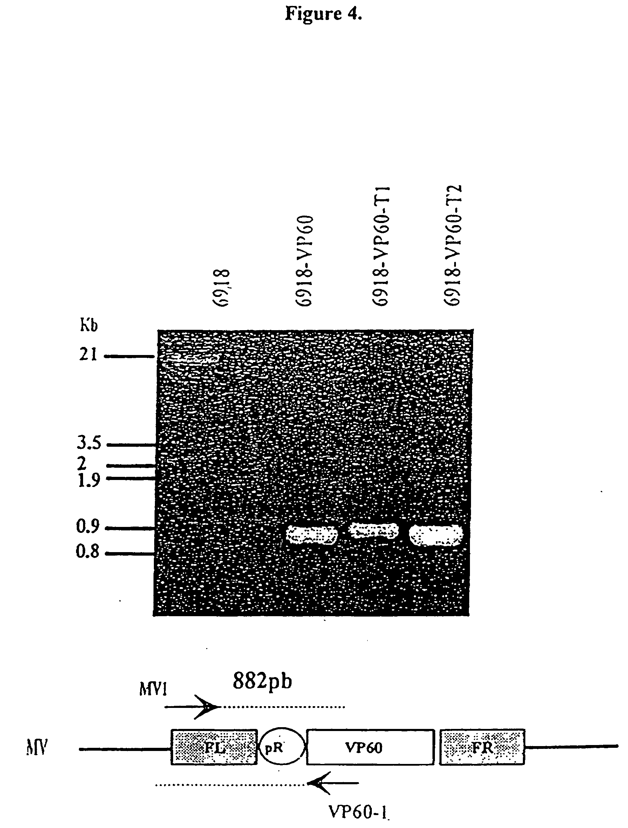

The double recombinants thereby obtained were amplified in RK-13 cell cultures and characterized. Fig. 4 shows the PCR results with the recombinants obtained in the 6918 strain.

With the aim of determining whether the product of the gene inserted in the MYX-VP60 recombinants expressed correctly and in sufficient quantity, expression experiments were performed on rabbit cell culturres infected with different recombinants. Two types of experiment have been carried out:
a) Immune-fluorescence. Specific rabbit serum against RHDV or specific VP60 monoclonals were used to detect the expression products in cell cultures infected by the myxoma-VP60 recombinants. The binding of specific antibodies was visualized with a second fluorescence antibody.
b) Immune-detection on nitrocellulose paper after electrophoresis. The protein extracts from cells infected by the MYX-VP60 recombinants were separated by electrophoresis in a polyacrylamide gel, and immediately afterward transferred to a nitrocellulose paper as described [Burnette, W.N., Analytical Biochemistry 112, 195-203 (1981)]. The presence of VP60 in this extract was determined using a specific rabbit hyper-immune serum against the RHDV or several monoclonal antibodies that specifically recognize the VP60 protein. The presence of the DA3 epitope in these same extracts was determined with an anti-DA3 monoclonal antibody.

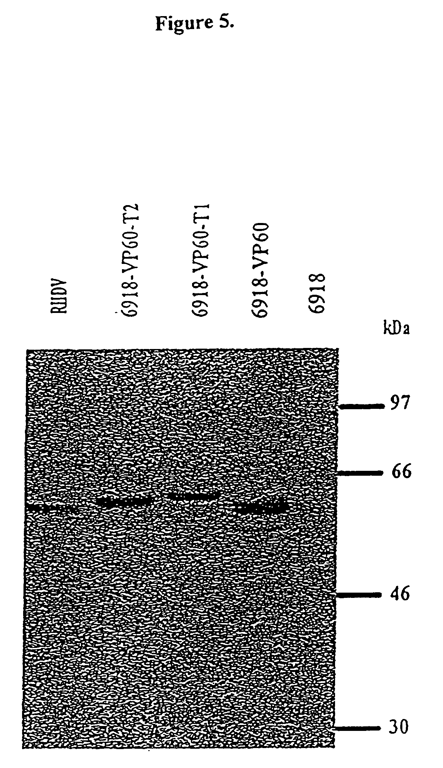

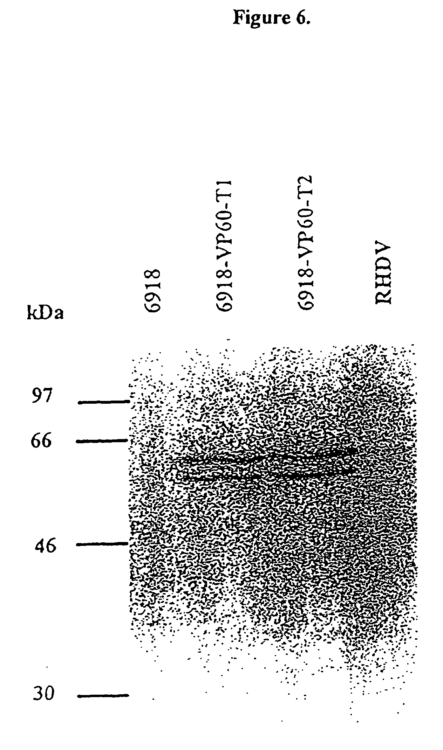

Figs. 5 and 6 show the results obtained with the recombinants derived from the 6918 strain. As can be observed, the cells infected with all the recombinants expressed a protein of approximately 60Kd recognized both by the anti-RHDV rabbit serum (Fig. 5) and by the anti-VP60 monoclonal antibodies (results not shown). This indicates that these recombinants correctly express the antigens contained within them. The electrophoretic mobility of the recombinant expression products that exclusively contain the VP60 sequence was similar to that of the VP60 obtained from the purified RHDV virus. While the recombinant expression products that further contained the DA3 epitope showed a slight increase of the apparent molecular weight.

The specific antibodies for the DA3 epitope (Fig. 6) recognized the expression product of those recombinants with VP60 versions that contained the marker, indicating that this epitope was expressed correctly in the two versions used.

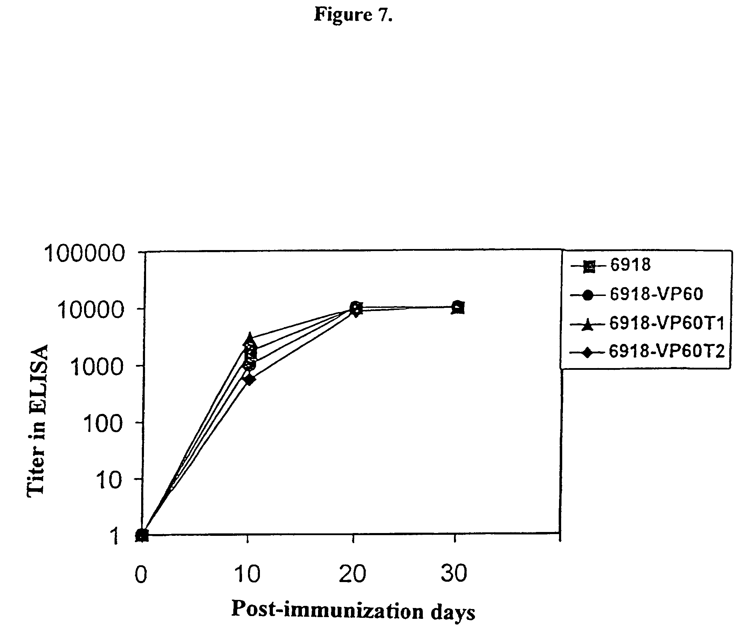

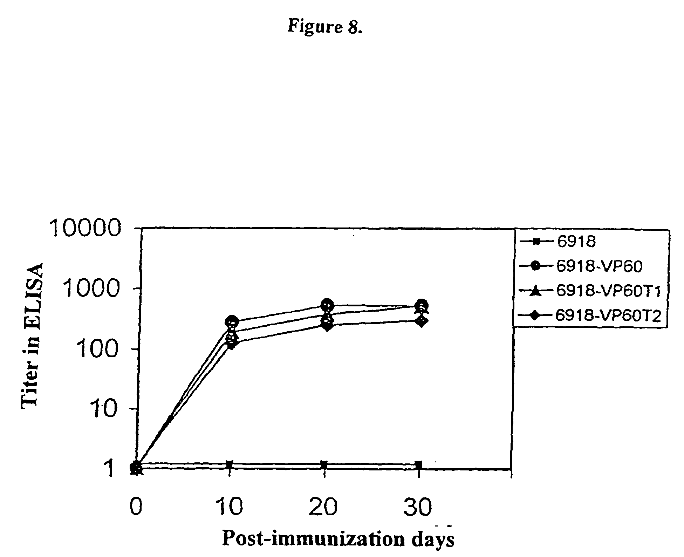

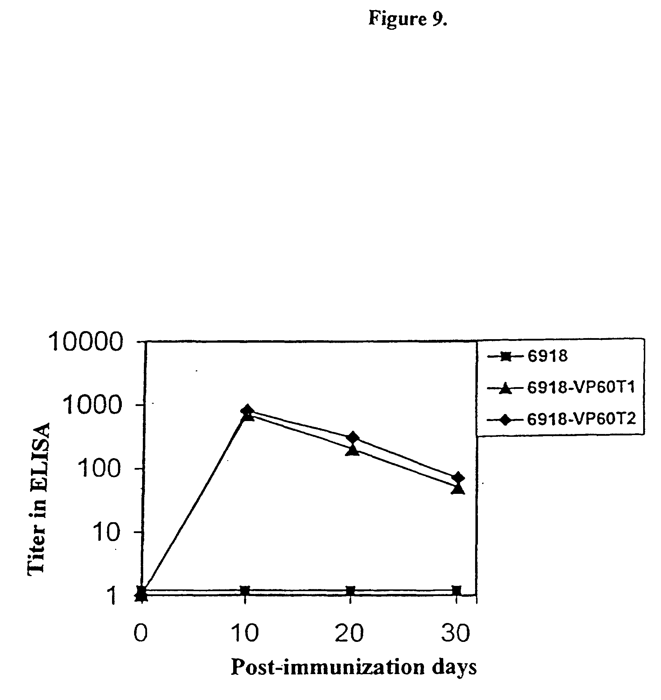

Subsequently, the immune response induced by the different myxoma-VP60 recombinants in domestic-wild hybrid rabbits was studied. To do this, two-month-old rabbits were immunized with 10⁴ plaque forming units (pfu) per animal of the recombinants constructed from the 6918 strains. The inoculation volume was 1.5 ml per rabbit and was administered by subcutaneous route. Control rabbits were also inoculated with the 6918 strain without insert under the same conditions. Serum samples were taken on days 0, 10, 20 and 30 of post-immunization. In the serum samples, the amount of anti-RHDV, anti-DA3 and anti-myxoma antibodies was determined by ELISA. For the ELISAs, 96 well polysorp micro-titer plates were coated with rabbit liver extracts infected with RHDV (0.1 µg/well) or cell extracts infected with myxoma (5 µg/well) or with TGEV (2 µg/well), and incubated over night at 37°C. This was followed by saturation using 5% skim-milk in PBS for 2 hours at 37°C. After this, the problem sera were added in different dilutions and these were incubated 1 hour at 37°C. Finally G protein peroxydase was added (1 hour at 37°C) in order to be developed afterwards with ABTS and to determine the optical density on an ELISA reader. The titer of the ELISA was defined as a the highest dilution where the optical density observed is two times greater as that of a negative control serum. The titer values of anti-RHDV, anti-DA3 and anti-myxoma antibodies induced by the different recombinants according to the post-immunization time are shown in Figs. 7, 8 and 9 respectively. As can be seen, at 10 days of post-immunization all the recombinants had induced the antibodies against the myxoma (Fig. 7) and against RHDV (Fig. 8). These ELISA titers continued increasing until 30 days of post-immunization, where they reached their maximum value. However, as expected, the myxoma virus strains without insert did not induce an anti-RHDV antibody response. The recombinants that contained the DA3 epitope induced in addition, a response of specific antibodies for the DA3 epitope, detectable from post-inoculation day 10 and onwards (Fig. 9).

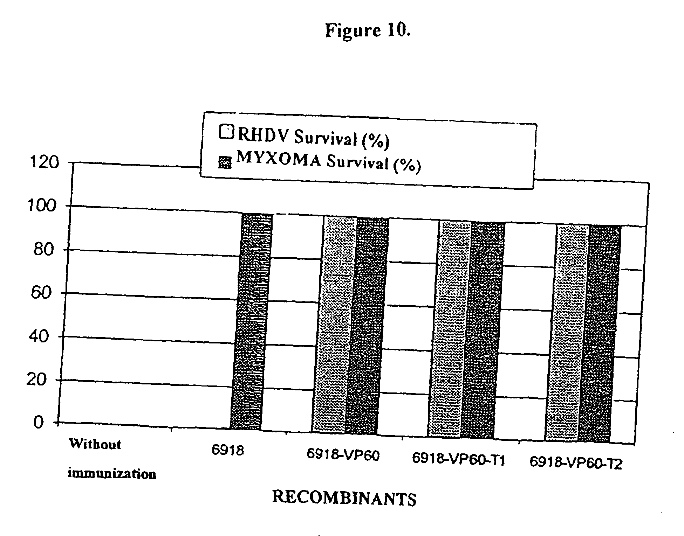

To determine whether the response induced by the myxoma-VP60 recombinants protected the animals against the *myxoma* and RHDV virus, rabbits were inoculated, immunized under the same conditions of the previous paragraph, with a lethal dosage of the Laussane strain of the *myxoma* virus (10³ pfu/animal) or of the RHDV AST/89 strain (10 LD50/animal), in a final volume of 1 ml per animal. The results of survival are presented in figure 10, where it is possible to observe that in the animals challenged with a highly pathogenic myxoma virus strain, the controls died in between 15 and 25 days, while the immunized animals remained alive without any type of disease symptoms. In the animals challenged with RHDV, all the controls died within 48 hours, while all the rabbits immunized with any of the recombinants remained alive and without any type of disease symptoms.

With the object of studying the eventual pathological damage produced through inoculation with myxoma-VP60 recombinants, two-month-old, wild-domestic, hybrid rabbits were immunized with 10⁴ plaque forming units (pfu) per animal of the recombinants constructed from the 6918 strain in a final volume of 1.5 ml per rabbit and administered via subcutaneous route. The clinical evolution of these animals was followed during the following six weeks and the results have been depicted in table 2.

**Table 2.**

| Evolution of the damage produced by the 6918 strain and its recombinants. | | | | | | | | | | | | |
|---|---|---|---|---|---|---|---|---|---|---|---|---|
| Post-inoculation days | | | | | | | | | | | | |
| Virus | Rabbit No. | 2 | 4 | 6 | 8 | 10 | 12 | 14 | 16 | 18 | 20 | Days 22 to 36 |
| | 1 | --- | --- | NP | NP | NP | NP | NP | NP | R | --- | --- |
| 6918 | 2 | -- | --- | --- | NP | NP | NP | NP | NP | R | --- | --- |
| | 3 | --- | --- | NP | NP | NP | NP | NP | R | --- | --- | --- |
| | 1 | --- | --- | NP | NP | NP | NP | NP | NP | R | --- | --- |
| 6918-VP60 | 2 | --- | --- | NP | NP | NP | NP | NP | NP | NP | R | --- |
| | 3 | --- | --- | NP | NP | NP | NP | NP | NP | R | --- | --- |
| | 1 | --- | --- | NP | NP | NP | NP | NP | NP | R | --- | --- |
| 6918-VP60-T1 | 2 | --- | --- | --- | NP | NP | NP | NP | NP | NP | R | --- |
| | 3 | --- | --- | NP | NP | NP | NP | NP | R | --- | --- | --- |
| | 1 | --- | --- | NP | NP | NP | NP | NP | NP | R | --- | --- |
| 6918-VP60-T2 | 2 | --- | --- | NP | NP | NP | NP | NP | R⁻ | --- | --- | --- |
| | 3 | --- | --- | NP | NP | NP | NP | NP | R | --- | --- | --- |
| ---: absence of damage; NP: Nodule at inoculation point; R: recovered | | | | | | | | | | | | |

As can be seen in table 2, the rabbits inoculated with 6918-VP60 (with or without the DA3 marker) showed very slight symptoms characterized by the apparition of a small nodule at the inoculation point and sporadically at the base of the ears, recovering totally within 15-20 days of post-infection.

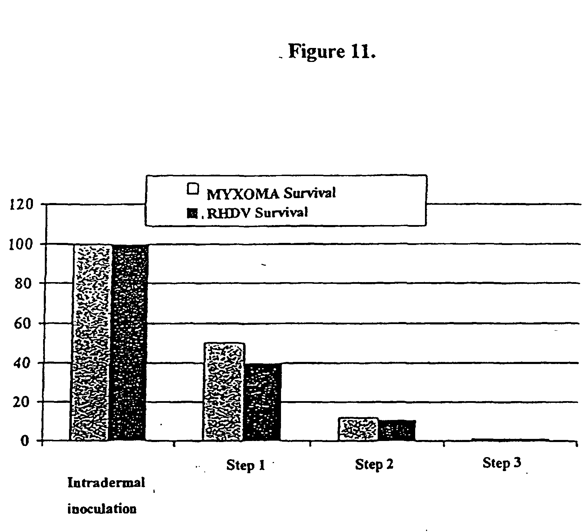

In view of the previous results the 6918-VP60-T2 vaccine virus was selected, since it is completely non pathogenic and induces a positive immune response capable of protecting against the two diseases, for study of its capacity for inducing protection via transmission through physical contact or fleas. In order to study transmission through physical contact, two-month-old rabbits were separated into 4 groups (A, B, C and D) of 5 rabbits each. The rabbits in group A were inoculated with 10⁴ pfu/animal of the 6918-VP60-T2 vaccine virus and at five days of post-inoculation they were placed in contact (placing them all in the same cage) with the group B rabbits, keeping them together for 7 days (designating this transmission Step 1). At the end of this time the group B animals were separated and placed in contact with the group C rabbits for another 7 days (Step 2). At the end of these 7 days, the group C rabbits were separated and placed in contact with the group D rabbits for another 7 days (Step 3). 30 days after inoculation (group A) or after transmission (groups B, C and D) the induced protection capacity in these animals against the two diseases (myxomatosis and RHDV) was determined. The protection trials were performed as described in the preceding paragraphs, studying the survival capacity after inoculation with a lethal dosage of the *myxoma* virus Laussane strain (10³ pfu/animal) or of the RHDV AST/89 strain (10 DL50/animal), in a final volume of 1 ml per animal. The results of survival are presented in Figure 11, where it can be seen that the transmission capacity through contact in Step 1 is 60%, since 60% of the animals have acquired protection capacity against the two diseases. However, in the following steps the transmission capacity diminishes and in step 3 the protection has been transmitted to none of the animals and the survival rate is 0% (all the animals die).

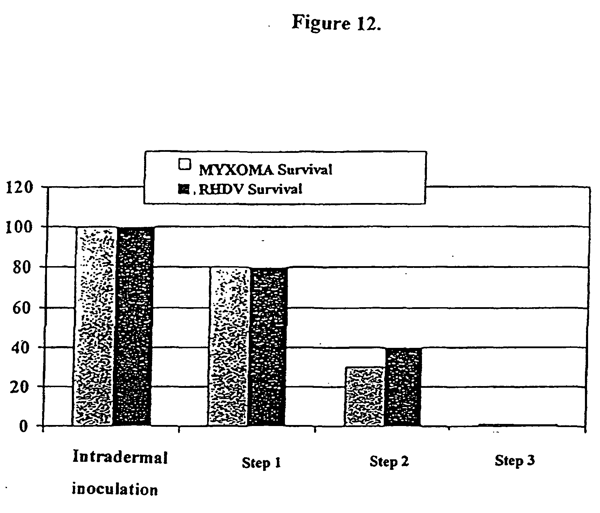

Similar experiments to those previously described were performed to determine the transmission capacity of the 6918-VP60-T2 vaccine virus through fleas. To do this, special cages were used, containing two dwelling spaces separated by two sets of metallic bars which impeded all contact between rabbits from one to the other space but allowed the passage of fleas from one space to another. The experiment was performed following the procedure described in the previous paragraph. The group A rabbits were inoculated with 10⁴ pfu/animal of the 6918-VP60-T2 vaccine virus and at day 5 post-inoculation 200 fleas were added to the cage. 48 hours later (post-inoculation day 7) the group B rabbits were placed in the cage, but in the separate space, for a further 7 days (Step 1). After this time, the group A rabbits were substituted for the group C rabbits, which were kept in the cage for 7 days more (Step 2). Following this, the group B rabbits were substituted for the group D rabbits (Step 3). 30 days after inoculation (group A) or after transmission (groups B, C and D) the induced protection capacity in these animals against the two diseases (myxomatosis and RHDV) was determined. The results of survival are presented in figure 12, where it can be seen that the transmission capacity through fleas in Step 1 is 80% since 80% of the animals have acquired protection capacity against the two diseases. However, in the following steps the transmission capacity diminishes and in Step 3 the protection has been transmitted to none of the animals and survival rate is 0% (none of the animals survive super-infection with myxoma or RHDV).

When the transmission experiments are performed in conditions allowing contact between animals and in the presence of fleas, the results are similar to those previously described but with higher protection levels, reaching 100% survival in Step 1. However, the transmission capacity is still limited to two steps and in Step 3 survival rate is 0%.

Throughout the transmission experiments, the clinical evolution of all the animals was followed. The rabbits inoculated intradermally with 6918-VP60-T2 showed mild symptoms similar to that observed in previous experiments and described in table 1. However, in the animals vaccinated by transmission, in the absence or presence of fleas, no clinical symptoms were observed except in some isolated animal in Step 1 in which conjunctivitis was observed which disappeared in the following three or four days.

## Claims

1. The recombinant myxoma virus deposited the 6^{th} March 1998 at the CNCM with deposit number I-1990.

2. The use of the virus with deposit number I-1990 for the preparation of combined vaccines against myxomatosis and rabbit hemorrhagic disease.

3. The combined vaccines against myxomatosis and rabbit hemorrhagic disease that comprise the virus with deposit number I-1990.

4. Vaccines according to claim 3, that comprise a vaccine dosage per animal of between 10² and 10⁵ plaque forming units of the I-1990 virus.

5. The vaccines according to any of the claims 3 and 4, capable of being administered through intradermal, subcutaneous, or oral routes.

6. A method for obtaining recombinant *myxoma* virus, attenuated and self-propagating in a controlled manner, **characterized in that** it comprises the following stages:
a) selecting and isolating a *myxoma* virus exhibiting a null or highly reduced mortality and possessing a transmission capacity of no greater than 3 transmission steps;
b) inserting in an intergenic region of its genome, through conventional DNA-recombinant techniques, one or more nucleotide sequences capable of expressing antigen polypeptides or proteins against microorganisms that produce infectious rabbit diseases and a promoter controlling such expression, and wherein the insertion is not affecting the transmission capacity of the virus,
c) cloning and replicating the virus obtained.

7. A method according to claim 6, **characterized in that** the nucleotide sequences are inserted into the intergenic region situated between the thymidine kinase gene and the MF8a gene, in such a manner that the insertion respects the regulating sequences of the TK gene, and the sequences previous to the ATG start codon of the MF8a gene.

8. A method according to any of the claims 6 and 7 **characterized in that**, in addition, a second nucleotide sequence is inserted, that encodes a second antigen polypeptide the detection of which allows differentiation between vaccinated and unvaccinated animals.

9. A method according to claim 6, **characterized in that** the nucleotide sequence capable of expressing antigen polypeptides or proteins for microorganisms that produce infectious rabbit diseases is a sequence that expresses an antigen protein of the rabbit hemorrhagic disease virus (RHDV).

10. A method according to claim 9, **characterized in that** the nucleotide sequence expresses the RHDV protein VP60.

11. A method according to claim 6, **characterized in that** the promoter controlling the expression is an early/late synthetic promoter.

12. A method according to claim 8, **characterized in that** the sequence that expresses the detecting antigen polypeptide is the epitope sequence of the N protein of the porcine gastroenteritis virus.

13. A method according to any of the claims 6 to 12, **characterized in that** the virus obtained is that corresponding to the deposit I-1990.

## Patentansprüche

1. Der am 6. März 1998 in der CMCN mit der Depotnummer I-1990 rekombinante Myxoma-Genvirus.

2. Die Verwendung des Virus mit der Depotnummer I-1990 für die Vorbereitung von Kombi-Impfstoffen gegen Myxomatose und die hämorrhagische Kaninchenkrankheit.

3. Kombi-Impfstoffe gegen Myxomatose und die hämorrhagische Kaninchenkrankheit, die den Virus mit der Depotnummer I-1990 enthalten.

4. Impfstoffe nach Anspruch 3, welche eine Impfstoffdosis zwischen 10² und 10⁵ Einheiten pro Tier enthalten, die den I-1990-Virenbelag bilden.

5. Impfstoffe nach einem der Ansprüche 3 und 4, die intradermisch, subkutan oder oral verabreicht werden können.

6. Ein Verfahren zum Erhalt eines gentechnisch abgeschwächten *Myxoma*-Virus, der in der Lage ist, sich in kontrollierter Weise selbst zu verbreiten und **dadurch gekennzeichnet, dass** er folgende Phasen durchläuft:
a) Auswahl und Isolation eines *Myxoma*-Virus, der keine oder nur eine sehr geringe Sterblichkeit aufweist und über eine Übertragungsfähigkeit von nicht mehr als 3 Übertragungsphasen verfügt;
b) Ansatz einer oder mehrerer Nukleotidsequenzen in einem intergenen Bereich seines Genoms durch konventionelle rekombinante DNA-Gentechniken, die Polypeptide oder Antigen-Proteine gegen Mikroorganismen anzeigen können, welche Infektionskrankheiten beim Kaninchen verursachen, und einen Träger zur Kontrolle dieser Erkennung, und bei dem der Ansatz die Übertragungsfähigkeit des Virus nicht beeinflusst,
c) Klonen und Replizieren des erhaltenen Virus.

7. Ein Verfahren nach Anspruch 6, **dadurch gekennzeichnet, dass** die Nukleotidsequenzen im intergenen Bereich zwischen dem Thymidin-Kinase-Gen und dem MF8a-Gen angesetzt werden, so dass der Ansatz die Regulierungssequenzen des TK-Gens und die Sequenzen vor dem Startkodon (ATG) des MF8a-Gens beachtet.

8. Ein Verfahren nach einem der Ansprüche 6 und 7, **dadurch gekennzeichnet, dass** zusätzlich eine zweite Nukleotidsequenz angesetzt wird, die ein zweites Antigen-Polypeptid kodifiziert, durch dessen Erkennung man zwischen geimpften und nicht geimpften Tieren unterscheiden kann.

9. Ein Verfahren nach Anspruch 6, **dadurch gekennzeichnet, dass** die Nukleotidsequenz, die in der Lage ist, Polypeptide oder Antigen-Proteine für Mikroorganismen anzuzeigen, die Infektionskrankheiten beim Kaninchen verursachen, eine Sequenz ist, die ein Antigen-Protein des Virus der hämorrhagischen Kaninchenseuche (RHDV) erkennen läßt.

10. Ein Verfahren nach Anspruch 9, **dadurch gekennzeichnet, dass** die Nukleotidsequenz das VP60-Protein des RHDV erkennen läßt.

11. Ein Verfahren nach Anspruch 6, **dadurch gekennzeichnet, dass** der die Erkennung kontrollierende Träger ein früher oder später synthetischer Träger ist.

12. Ein Verfahren nach Anspruch 8, **dadurch gekennzeichnet, dass** die Sequenz, welche den Antigen-Polypeptid-Detektor erkennen läßt, die epitope Sequenz des N-Proteins des Schweinegastroenteritis-Virus ist.

13. Ein Verfahren nach einem der Ansprüche 6 bis 12, **dadurch gekennzeichnet, dass** der erhaltene Virus dem Virus im Depot I-1990 entspricht.

## Revendications

1. Myxoma virus recombinant déposé le 6 mars 1998 auprès du CNCM sous le numéro de dépôt I-1990.

2. Utilisation du virus portant le numéro de dépôt I-1990 pour préparer des vaccins combinés contre la myxomatose et la maladie hémorragique du lapin.

3. Vaccins combinés contre la myxomatose et la maladie hémorragique du lapin, qui comprennent le virus portant le numéro de dépôt I-1990.

4. Vaccins selon la revendication 3, qui comprennent une dose de vaccin par animal comprise entre 10² et 10⁵ unités formant des plages du virus I-1990.

5. Vaccins selon l'une quelconque des revendications 3 et 4, capables d'être administrés par voie intradermique, sous-cutanée ou orale.

6. Procédé d'obtention de myxoma virus recombinant, atténué et présentant une auto-prolifération régulée, **caractérisé en ce qu'**il comprend les étapes suivantes :
a) sélection et isolement d'un myxoma virus présentant une mortalité nulle ou fortement réduite et possédant une capacité de transmission non supérieure à 3 étapes de transmission ;
b) insertion, dans une région intergénique de son génome, par des techniques classiques de recombinaison d'ADN, d'une ou plusieurs séquences nucléotidiques capables d'exprimer des polypeptides ou protéines antigéniques contre des microorganismes à l'origine des maladies infectieuses du lapin, et d'un promoteur régulant cette expression, l'insertion n'affectant pas la capacité de transmission du virus ;
c) clonage et réplication du virus obtenu.

7. Procédé selon la revendication 6, **caractérisé en ce que** les séquences nucléotidiques sont insérées dans la région intergénique située entre le gène de la thymidine-kinase et le gène MF8a de telle manière que l'insertion respecte les séquences régulatrices du gène TK, et les séquences qui précèdent le codon non-sens ATG du gène MF8a.

8. Procédé selon l'une quelconque des revendications 6 et 7, **caractérisé en ce que**, en outre, une deuxième séquence nucléotidique est insérée, qui code pour un deuxième polypeptide antigénique dont la détection permet une différentiation des animaux vaccinés et des animaux non vaccinés.

9. Procédé selon la revendication 6, **caractérisé en ce que** la séquence nucléotidique capable d'exprimer des polypeptides ou protéines antigéniques pour des microorganismes à l'origine des maladies infectieuses du lapin est une séquence qui exprime une protéine antigénique du virus de la maladie hémorragique du lapin (RHDV).

10. Procédé selon la revendication 9, **caractérisé en ce que** la séquence nucléotidique exprime la protéine VP60 du RHDV.

11. Procédé selon la revendication 6, **caractérisé en ce que** le promoteur qui régule l'expression est un promoteur synthétique précoce/tardif.

12. Procédé selon la revendication 8, **caractérisé en ce que** la séquence qui exprime le polypeptide antigénique de détection est la séquence de l'épitope de la protéine N du virus de la gastroentérite porcine.

13. Procédé selon l'une quelconque des revendications 6 à 12, **caractérisé en ce que** le virus obtenu est celui qui correspond au dépôt I-1990.


### SEQUENCE LISTING

<210> 1
   <211> 78
   <212> DNA
   <213> Artificial Sequence
<220>
   <223> Description of Artificial Sequence: Early/late promoter
<300>
   <303> Gene
   <304> 158
   <306> 157-162
   <307> 1995
<400> 1
   gatccatctc gagttattta tattccaaaa aaaaaaaata aaatttcaat ttttccatgg 60
   gctagcagat ctaagctt 78
<210> 2
   <211> 78
   <212> DNA
   <213> Artificial Sequence
<220>
   <223> Description of Artificial Sequence: Early/Late promoter
<300>
   <303> Gene
   <304> 158
   <306> 157-162
   <307> 1995
<400> 2
   gatcaagctt agatctgcta gcccatggaa aaattgaaat tttatttttt ttttttggaa 60
   tataaataac tcgagatg 78
<210> 3
   <211> 26
   <212> DNA
   <213> Artificial Sequence
<220>
   <223> Description of Artificial Sequence: PCR primers
<400> 3
   caccgaattc taggacccat gtttgc 26
<210> 4
   <211> 33
   <212> DNA
   <213> Artificial Sequence
<220>
   <223> Description of Artificial Sequence: PCR primers
<400> 4
   tttatttttc catggtttta aaaaaaataa cat 33
<210> 5
   <211> 33
   <212> DNA
   <213> Artificial Sequence
<220>
   <223> Description of Artificial Sequence: PCR primer
<400> 5
   tacaactcga ggtgtggatc ctgttatttt ttt 33
<210> 6
   <211> 25
   <212> DNA
   <213> Artificial Sequence
<220>
   <223> Description of Artificial Sequence: PCR primer
<400> 6
   tataagacac gtcgacgtca ctgaa 25
<210> 7
   <211> 35
   <212> DNA
   <213> Artificial Sequence
<220>
   <223> Description of Artificial Sequence: PCR primer
<400> 7
   cgggctcgag cactaattcc aaacccaccc gcttt 35
<210> 8
   <211> 35
   <212> DNA
   <213> Artificial Sequence
<220>
   <223> Description of Artificial Sequence: PCR primer
<400> 8
   attggagctc gcctgaagtt aaaaagaaca acgcc 35
<210> 9
   <211> 23
   <212> DNA
   <213> Artificial Sequence
<220>
   <223> Description of Artificial Sequence: PCR primer
<400> 9
   gagcacgaat tcaaacccac ccg 23
<210> 10
   <211> 25
   <212> DNA
   <213> Artificial Sequence
<220>
   <223> Description of Artificial Sequence: PCR primer
<400> 10
   cgcctgaatt caaaaagaac aacgc 25
<210> 11
   <211> 25
   <212> DNA
   <213> Artificial Sequence
<220>
   <223> Description of Artificial Sequence: PCR primer
<400> 11
   ccagtccgat gagatctgac atagg 25
<210> 12
   <211> 26
   <212> DNA
   <213> Artificial Sequence
<220>
   <223> Description of Artificial Sequence: PCR primer
<400> 12
   caccgaattc taggacccat gtttgc 26
<210> 13
   <211> 26
   <212> DNA
   <213> Artificial Sequence
<220>
   <223> Description of Artificial Sequence: PCR primer
<400> 13
   gaaaacatca ttataataaa agttcg 26